# EUROPEAN PATENT SPECIFICATION

(11) **EP 0 904 044 B1**
(45) Date of publication and mention of the grant of the patent: **09.08.2000**
(21) Application number: 97915582.7
(22) Date of filing: 01.04.1997
(51) Int. Cl.: A61K 7/06, A61K 7/032

(54) **METHOD AND COMPOSITION FOR THE CURLING OF EYE LASHES**
VERFAHREN UND MITTEL ZUM VERFORMEN VON WIMPERN
PROCEDE ET COMPOSITION SERVANT A RECOURBER LES CILS

(30) Priority: 02.04.1996 GB 9606936
(43) Date of publication of application: 31.03.1999
(73) Proprietor: Collis, Joanna, Rudgewick, Horsham RH12 3AS (GB); Hose, Sara-Jane, Rudgewick, Horsham RH12 3AS (GB)
(72) Inventor: Collis, Joanna, Rudgewick, Horsham RH12 3AS (GB); Hose, Sara-Jane, Rudgewick, Horsham RH12 3AS (GB)
(74) Representative: Selby-Lowndes, Guy Francis Charles
(86) International application number: GB9700932
(87) International publication number: WO9736569

(56) References cited:
- WO-A-91/15187
- US-A- 4 988 502
- US-A- 5 384 118
- SOAP, COSMETICS, CHEMICAL SPECIALTIES, vol. 69, no. 3, 1993, NEW YORK (USA), page 73 XP000353618 "Mascara"
- CHEMICAL ABSTRACTS, vol. 88, no. 2, 9 January 1978 Columbus, Ohio, US; abstract no. 11741, TSUKAMOTO, AKIRA ET AL: "Hair conditioners" XP002036292 & JP 52 105 227 A (MATSUSHITA ELECTRIC WORKS, LTD., JAPAN)
- CHEMICAL ABSTRACTS, vol. 115, no. 20, 18 November 1991 Columbus, Ohio, US; abstract no. 214524, MAEDA, NORIKO ET AL: "Transparent hair preparation containing esthetic stereopattern" XP002036293 & JP 03 178 917 A (POLA CHEMICAL INDUSTRIES, INC., JAPAN)
- CHEMICAL ABSTRACTS, vol. 106, no. 10, 9 March 1987 Columbus, Ohio, US; abstract no. 72693, KANAYAMA, HIROSHI ET AL: "Gel hair conditioners containing vinylpyrrolidone polymers, carboxyvinyl polymer, basic substances, alcohols, and oils" XP002036294 & JP 61 229 814 A (KANEBO, LTD., JAPAN)

## Description

This invention relates to a method of curling eye lashes that can be carried out without professional assistance and to a composition for use in the method.

The curling of eye lashes raises problems that do not occur with hair as the proximity to the eye precludes the use of most chemical methods and makes the use of heated tongs hazardous. Professional beauty saloons provide an eye lash curling service at considerable cost. As the lifespan of an eye lash is approximately thirty days the treatment must be carried out frequently.

US-A-5 384 118 discloses a hairdressing gel composition which includes a blend of measured quantities of water, alcohol, PVP/VA copolymer, glycerin, surfactants, tetrasodium EDTA, carbomer 940, triethanolamine, coconut oil, lanolin and vitamin E. A stated object of the gel is that it precludes hair from becoming stiff.

US-A-4 988 502 discloses a mascara composition. The composition comprises a hydrophobic pigment, a film-forming resin, a water-dispersible thickening agent, a humectant and water. The pigment is rendered hydrophobic by treatment with lecithin. The preferred and exemplified film-forming resin is polyvinylalcohol.

The present invention provides a method of curling eye lashes which can be carried out with minimal hazard to the eye and which is suitable for home use together with a composition for use in the method.

According to the present invention there is provided a setting lotion comprising an aqueous alcoholic mixture containing a dissolved resin and in gel form, characterised in that the resin is a polyvinylpyrrolidone/vinylacetate copolymer and in that it is dissolved in a mixture of ethanol and water in proportions from 1/2.5 to 1/3 parts by weight.

There is also provided a method of curling eye lashes comprising applying the above setting lotion in gel form to a row of eye lashes, holding the lashes in the required shape using tongs until the setting action is complete, thereafter releasing the lashes and removing surplus setting lotion.

The setting lotion in gel form is preferably applied using a small brush. The preferred brush has bristles at one end extending radially from a straight handle formed from twisted wire, wood or a synthetic polymer. The brush may comprise part of the container in which the lotion is supplied. Preferably while applying the lotion to the eye and upper cheek is protected by an absorbent pad such as a gauze pad.

Setting takes place after the lotion has been applied through evaporation of the water and alcohol. A thin film of resin remains which is found to have sufficient strength to maintain each eye lash in the desired shape for long periods. The resin is a synthetic polymer with appreciable elasticity. This ensures that the coating of resin on each lash does not flake in the event that the lash is flexed. The concentration, solvent composition and resin type control the thickness of the resin coating deposited on each lash. The flexibility of the resin coating may be increased by including one or more plasticisers which remain in the resin coating after the solvent mixture has evaporated.

The resin is a polyvinylpyrrolidone/vinyl acetate copolymer. The preferred plasticisers are polyethylene glycols.

In order to convert the lotion into a gel form suitable gelling agents are incorporated into the liquid so that it becomes non-Newtonian in flow, preferably thixotropic, so that the lotion can be applied easily with a brush to the lashes but does not flow off subsequently and remains in place while the solvents evaporate. A preferred gelling agent is an acidic carboxyvinyl polymer. such polymers may be mixed with the other components of the lotion in a slightly acidic liquor phase after which the pH is increased causing the dispersed polymer to form a gel.

The shape tends to be lost after washing the face or showering unless the eye lashes are covered with a protective layer of tape or similar water impermeable material. However the curling method may be repeated easily after ablutions have taken place. Eye make-up, such as mascara, may be applied after the lashes have been curled in the conventional manner.

Other components may be added to the mixture before gelling such as denaturants to discourage accidental or deliberate ingestion, perfume and colourants.

The solvent mixture is ethanol and water in proportions 1/2.5 to 1/3.5 parts by weight and contains 1 to 4 percent, more preferably 1.5 to 2.5 percent, by weight resin.

The invention will be illustrated by the following examples of gelled lotions used for setting eye lashes.

### Example 1

An eye lash gel was prepared having the following composition in percentage by weight:

| | |
|---|---|
| Ethanol | 30.0 |
| Luviskol VA 37 E | 2.0 |
| A.M.P. 100 | 0.5 |
| Carbopol Ultrez 10 polymer | 0.60 |
| Bitrex | 0.08 |
| Disodium EDTA | 0.05 |
| Nipaguard BPX | 0.05 |
| Deionised water to make 100 percent | |

Luviskol VA 37 E is a polyvinylpyrrolidone/vinylacetate (PVA/VA0 copolymer supplied as an approximately 50 % solution in ethanol.
A.M.P. 100 is aminopropanol.
Carbopol Ultrez 10 polymer is an acidic carboxyvinyl polymer gelling agent.
Bitrex is a denaturing agent
Nipaguard is a methyl paraben, 2-bromo-nitropan-1,3-diol, propylparaben and phenoxyethanol mixture.

The lotion was prepared in three stages as follows:

### STAGE 1

A first mixture was prepared containing:

| | |
|---|---|
| Ethanol | 27.0 g |
| Disodium EDTA | 0.05 g |
| Carbopol Ultrez 10 Polymer | 0.6 g |
| Deionised water | 65.5 g |

The mixture was rendered homogeneous by use of a high speed stirrer to disperse the Carbopol Ultrez polymer. 15

### STAGE 2

A second mixture was prepared containing:

| | |
|---|---|
| Ethanol | 3.0 g |
| Luviskol VA 37 E | 2.0 g |
| Nipaguard BPX | 0.05 G |
| Bitrex solution | 0.08 g |

In order to obtain homogeneous mixing the Luviskol polymer, supplied as a 50% solution in ethanol, was first mixed with the ethanol component. The other components were added subsequently with stirring.

### STAGE 3

A third mixture was prepared containing:

| | |
|---|---|
| A.M.P. 100 | 0.5 g |
| Deionised water | 1.0 g |

The mixtures from stages 1 and two were stirred together to form a homogeneous combination. This was allowed to stand after mixing to enable any entrained air to escape. After deaeration the mixture from stage 3 was added to raise the pH and cause and the Carbopol Ultra dispersion to form a gel.

The final pH was in the range 6.5 to 7.2.

### Example 2

A high strength eye lash gel was prepared having the following composition in percentage by weight:

| | |
|---|---|
| Ethanol | 25.2 |
| Luviskol VA 37 E | 4.0 |
| A.M.P. 100 | 0.65 |
| Carbopol Ultrez 10 polymer | 0.60 |
| Bitrex | 0.08 |
| Disodium EDTA | 0.05 |
| Nipaguard BPX | 0.05 |
| Deionised water to make 100 percent | |

The components were mixed in three stages in the manner described in Example 1 to obtain the product which contained approximately two percent by weight PVA/VA resin.

The gelled lotion was applied to eye lashes and was allowed to dry for between 20 and 60 seconds. The time depended on the ambient temperature and humidity. After treatment the eye lashes were curled using tongs and the eye lashes retained the curl. It was possible to enhance the appearance of the eye lashes in a conventional manner by the application of mascara.

The gelled setting lotion is preferably packaged in jars or tubes. The lotion may be removed from the former by use of the applicator brush or in the case of the latter it may be squeezed directly on to the bristles. In a preferred form a the setting lotion is stored in a substantially tubular container enclosed with a cap carrying a brush whose stem and head is fits into the container.

## Claims

1. A setting lotion comprising an aqueous alcoholic mixture containinq a dissolved resin and in gel form, characterised in that the resin is a polyvinylpyrrolidone/vinylacetate copolymer and in that it is dissolved in a mixture of ethanol and water in proportions from 1/2.5 to 1/3 parts by weight.

2. The setting lotion according to claim 1, characterised in that the dissolved resin is present in an amount from 1 to 4 percent by weight.

3. The setting lotion according to claim 2, characterised in that the dissolved resin is present in an amount from 1.5 to 2.5 percent by weight.

4. The setting lotion according to any of the preceding claims, characterised in that the gelling agent is an acidic carboxyvinyl polymer.

5. The setting lotion according to any of the preceding claims, characterised in that it contains a plasticizer for the resin.

6. A method of curling eye lashes characterised in that a setting lotion in gel form according to any of the claims 1 to 5 is applied to a row of eye lashes, holding the lashes in the required shape using tongs until the setting action is complete, thereafter releasing the lashes and removing surplus setting lotion.

## Patentansprüche

1. Haarfestiger, umfassend ein wäßriges alkoholisches Gemisch, das ein gelöstes Harz enthält und in Gelform vorliegt, dadurch gekennzeichnet, daß der Harz ein Copolymer aus Polyvinyl-Pyrrolidon und Vinylacetat ist, und dadurch, daß er in einem Gemisch aus Ethanol und Wasser in Proportionen von 1:2,5 bis 1:3 Gewichtsteilen aufgelöst ist.

2. Haarfestiger nach Anspruch 1, dadurch gekennzeichnet, daß der aufgelöste Harz in einer Menge von 1 bis 4 Gew.-% vorliegt.

3. Haarfestiger nach Anspruch 2, dadurch gekennzeichnet, daß der aufgelöste Harz in einer Menge von 1,5 bis 2,5 Gew.-% vorliegt.

4. Haarfestiger nach einem der vorherigen Ansprüche, dadurch gekennzeichnet, daß das Geliermittel ein azides Carboxyvinyl-Polymer ist.

5. Haarfestiger nach einem der vorherigen Ansprüche, dadurch gekennzeichnet, daß er ein Plastiziermittel für den Harz aufweist.

6. Verfahren zum Formen von Wimpern, dadurch gekennzeichnet, daß ein Haarfestiger in Gelform nach einem der Ansprüche 1 bis 5 auf eine Reihe Wimpern aufgebracht wird, die Wimpern mit einem Greifer in der gewünschten Form gehalten werden, bis der Verfestigungsvorgang abgeschlossen ist, und die Wimpern danach losgelassen werden und überschüssiger Haarfestiger entfernt wird.

## Revendications

1. Une lotion de fixation englobant un mélange alcoolique aqueux contenu dans une résine dissoute et sous forme de gel, caractérisée en ce que la résine est un copolymère polyvinylpyrrolidone/acétate de vinyle et en ce qu'il est dissous dans un mélange d'éthanol et d'eau dans des proportions comprises entre 1/2,5 et 1/3 parties en poids.

2. La lotion de fixation selon la revendication 1, caractérisée en ce que la résine dissoute est présente en une quantité de 1 à 4 pour cent en poids.

3. La lotion de fixation selon la revendication 2, caractérisée en ce que la résine dissoute est présente en une quantité de 1,5 à 2,5 pour cent en poids.

4. La lotion de fixation selon l'une quelconque des revendications précédentes, caractérisée en ce que l'agent gélifiant est un polymère carboxyvinylique acide.

5. La lotion de fixation selon l'une quelconque des revendications précédentes, caractérisée en ce qu'elle contient un plastifiant pour la résine.

6. Un procédé servant à recourber les cils, caractérisé en ce qu'une lotion de fixation sous forme d'un gel selon l'une quelconque des revendications 1 à 5 est appliquée à une rangée de cils, en maintenant les cils en la forme souhaitée au moyen de pinces à cils jusqu'à ce que l'effet de fixation soit complet, puis en relâchant les cils et en retirant la lotion de fixation en excédent.
